(19) Europäisches Patentamt
European Patent Office
Office européen des brevets

(11) Publication number: **0 056 994**
**B1**

# (12) EUROPEAN PATENT SPECIFICATION

(45) Date of publication of patent specification: **28.11.84**

(51) Int. Cl.[3]: **C 07 C 69/36,** C 07 C 67/36

(21) Application number: **82100447.0**

(22) Date of filing: **22.01.82**

## (54) Process for preparation of oxalic acid diesters.

(30) Priority: **23.01.81 JP 7910/81**

(43) Date of publication of application:
**04.08.82 Bulletin 82/31**

(45) Publication of the grant of the patent:
**28.11.84 Bulletin 84/48**

(84) Designated Contracting States:
**BE DE FR GB IT NL**

(56) References cited:
**DE-B-1 518 876**
**GB-A-2 025 950**

**Chemical Abstracts vol. 93, no. 22, 1980 Columbus, Ohio, USA TOKYO SHIBAUR ELECTRIC "Gas sensor" page 719, column 1, abstract no. 214986v**

(73) Proprietor: **Ube Industries, Ltd.**
**12-32, Nishihonmachi 1-chome**
**Ube-shi, Yamaguchi-ken (JP)**

(72) Inventor: **Miyazaki, Haruhiko**
**820-14, Nishiube-cho**
**Ube-shi Yamaguchi-ken (JP)**
Inventor: **Shiomi, Yasushi**
**635-5, Yoshida Nishikiwa-ku**
**Ube-shi Yamaguchi-ken (JP)**
Inventor: **Fujitus, Satoru**
**1360-7, Kagawa**
**Yamaguchi-shi Yamaguchi-ken (JP)**
Inventor: **Masunaga, Katsuro**
**Nonaka-nishi**
**Ube-shi Yamagushi-ken (JP)**
Inventor: **Yanagisawa, Hiroshi**
**25-ku, Onda**
**Ube-shi Yamaguchi-ken (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**EP 0 056 994 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for preparing a diester of oxalic acid by the vapor (or gaseous) phase catalytic reaction of carbon monoxide with an ester of nitrous acid in the presence of a catalyst composed of a solid carrier and a platinum-group metal or a salt thereof supported on the carrier. According to this process, the diester of oxalic acid can be produced at a high space time yield and especially high selectivity with reduced formation of a carbonic acid diester and carbon dioxide as by-products. A further advantage of this process is that the amount of the platinum-group metal used can be decreased and it can be easily recovered.

The process for preparing a diester of oxalic acid by the vapor phase catalytic reaction of carbon monoxide with an ester of nitrous acid in the presence of a catalyst composed of a solid carrier and metallic palladium or a salt thereof supported on the carrier is known (U.S. Patent No. 4,229,591). This patent cites active carbon, alumina, silica, diatomaceous earth, pumice, zeolite and molecular sieves as examples of the solid carrier, but fails to disclose anything about a silicon carbide carrier.

Japanese Laid-Open Patent Publication No. 22666/1980 (published on February 18, 1980; corresponding to UK Patent Application 2025950A) discloses another process for the production of a diester of oxalic acid by a similar vapor phase catalytic reaction to that shown in the above U.S. Patent. The Japanese patent document exemplifies palladium, rhodium, iridium, platinum, gold and salts of these metals as ingredients of the catalyst, and silica, magnesium oxide, aluminum oxide, zinc oxide, $Cr_2O_3$ and pumice as examples of the carrier. It also exemplifies iron, copper and salts of these as a carrier which concurrently serves as a catalyst promoter. This patent publication does not give any reference to the use of a silicon carbide carrier.

The present inventors made investigations in order to provide a process for producing diesters of oxalic acid at a high space time yield and an especially high selectivity. These investigations have led to the discovery that the use of a silicon carbide carrier which is not at all recognized in the prior art brings about an improvement in the mechanical strength, stability and durability of the catalyst and the selectivity of the desired product.

In order to utilize expensive noble metals efficiently in carrier-supported noble metal catalysts, it is necessary to increase the degree of dispersion of the noble metals. Usually, therefore, carriers of a high specific surface area have been selected in the past. It has now been found in accordance with this invention that by using silicon carbide as a carrier, high selectivities can be achieved even when it has a low specific surface area, for example a specific area (measured by the BET method utilizing the adsorption of nitrogen gas) of about 0.001 to about 1 $m^2/g$. It has also been found that the use of the silicon carbide carrier increases the strength of the catalyst against heat changes, and makes the excellent selectivity of the desired product long-lasting.

The present inventors have also discovered that such a catalyst composed of silicon carbide as a carrier and a platinum-group metal or a salt thereof supported on the carrier is prepared especially preferably by impregnating the silicon carbide carrier with an aqueous solution of a water-soluble salt of the platinum-group metal, treating the impregnated silicon carbide carrier with an alkali, and subjecting the alkali-treated product to reducing treatment in the liquid or gaseous phase.

It is an object of this invention therefore to provide an improved process for the preparation of a diester of oxalic acid by vapor phase catalytic reaction.

The above and other objects and advantages of the invention will become more apparent from the following description.

The present invention concerns a process of the initially described type which is characterized in that said solid carrier is silicon carbide.

The catalyst used in the process of the invention is a catalyst composed of said silicon carbide carrier and a platinum-group metal or a salt thereof supported on the carrier.

Examples of the platinum-group metal are palladium, platinum, rhodium, ruthenium and iridium. These metals may, if desired, be used as a mixture of two or more. The use of palladium, either alone or with another metal of the platinum group, is preferred. The salt of the platinum-group metal include nitrates, sulfates, phosphates, halides, acetates, oxalates, and benzoates of the above exemplified metals.

The silicion carbide carrier used in this invention needs not to be chemically pure, and may include a small amount, for example up to 30% by weight, preferably up to 20% by weight, of impurities. Such impurities include, for example, silica, carbon, alumina, iron oxide, sodium oxide and potassium oxide.

The size and shape of silicon carbide may be properly selected. For example, it has a diameter of 1 to 10 mm, preferably 2 to 6 mm, and is in the form of spheres, pellets, etc.

The amount of the catalytic metal component to be supported can be properly selected. It is preferably 0.01 to 10% by weight, more preferably 0.1 to 2% by weight, calculated as the metal based on the weight of the silicon carbide carrier.

There is no restriction on the manner of supporting the catalytic metal component on the silicon carbide carrier, and any known means of supporting can be used. Preferably, however, the catalyst is prepared by impregnating silicon carbide with an aqueous solution

of a water-soluble salt of the platinum-group metal, treating the impregnated alumina carrier with an alkali, and then subjecting the product to reducing treatment in the liquid or gaseous phase.

Examples of the water-soluble salt are nitrates, sulfates, acetates, phosphates, chlorides and sodium complex salts of the above-exemplified platinum-group metals.

The impregnation may be performed by dipping silicon carbide in the aqueous solution of the platinum-group metal salt at a temperature of, for example, 0 to 90°C for a period of, for example 0.1 to 10 hours. If desired, it can be effected by spraying the aqueous solution of the platinum-group metal salt onto silicon carbide. Preferably, the aqueous solution of the platinum-group metal salt is a solution prepared by dissolving the platinum-group metal salt in an acidic aqueous solution containing 0.01 to 10% by weight of an acidic compound. The use of the acidic aqueous solution serves to aid in dissolving the platinum-group metal and to prevent the formation and precipitation of a hydroxide and oxide of the platinum-group metal by hydrolysis.

Compounds having the anionic group of the platinum-group metal salt can generally be used as such acidic compounds. Specific examples include mineral acids such as hydrochloric acid, nitric acid, sulfuric acid and phosphoric acid and organic acids such as acetic acid. These acidic compounds may, if desired, be used as a mixture of two or more.

The silicon carbide impregnated with the aqueous solution of the platinum-group metal salts is then separated, and if desired washed with water and then dried by, for example, air drying, vacuum drying or heat drying, after which it is subjected to the alkali treatment.

The alkali treatment can be effected by adding the silicon carbide impregnated with the aqueous solution of the platinum-group metal salt to an alkaline aqueous solution containing 0.5 to 10% by weight of an alkaline compound, and stirring the mixture at a temperature of, for example, 10 to 90°C for a period of, for example, 0.5 to 10 hours. Examples of the alkaline compound include the hydroxides and salts of alkali metals or alkaline earth metals, for example sodium hydroxide, potassium hydroxide, calcium hydroxide, barium hydroxide, sodium carbonate, sodium hydrogen carbonate and potassium carbonate. If desired, these alkaline compounds may be used as a mixture of two or more. There is no special limitation on the amount of the alkaline compound used. Preferably, it is 2 to 40 moles per mole of the platinum-group metal salt.

After the alkali treatment, the product is optionally washed with water, etc. and dried. The product is then treated with a reducing agent in the liquid or gaseous phase to form the final catalyst.

The liquid-phase reduction is carried out by using such reducing agents as hydrazine, formaldehyde, sodium formate and formic acid. Specifically, it can be carried out by adding the alkali-treated product to an aqueous solution of the reducing agent in a concentration of 0.5 to 10% by weight, and stirring the mixture at a temperature of e.g. 10 to 50°C for a period of, e.g. 0.5 to 10 hours.

The alkaline-treated product may be added directly to the aqueous solution of the reducing agent in performing the reduction. It is more effective, however, to separate the alkali-treated solid product by a solid-liquid separating procedure such as filtration or decantation, wash and dry it in the manner described above, and then add the dried product to the aqueous solution of the reducing agent in the liquid phase.

Examples of reducing agents suitable for use in the gaseous phase reduction are hydrogen, carbon monoxide and ammonia. These reducing agents may be used after being diluted with inert gases such as nitrogen or carbon dioxide. The gaseous phase reduction can be carried out by passing the gaseous reducing agent through the alkali-treated product at a temperature of, for example, 50 to 500°C for a period of e.g. 1 to 10 hours.

The starting gases used in the synthesis of diesters of oxalic acid in accordance with the process of this invention are carbon monoxide and a nitrous acid ester, and as required, may also include an alcohol, a nitrogen oxide, etc. to be described hereinbelow. In any case, the starting gases include carbon monoxide which is effective for the aforesaid reducing treatment. Thus, the gaseous phase reduction of the alkali-treated product may also be effected by a procedure which comprises filling the alkali-treated product into a reactor for the synthesis of a diester of oxalic acid, and prior to the synthesis of the diester of oxalic acid, treating it with a starting gaseous mixture of carbon monoxide and a nitrous acid ester optionally containing an alcohol, a nitrogen oxide, etc.

The final desired catalyst can be formed by performing the reduction in the above manner. In the case of the liquid reduction, the treated product may be washed with water, etc. and dried.

According to this process of this invention, carbon monoxide is reacted with an ester of nitrous acid in the vapor phase in the presence of the resulting catalyst composed of silicon carbide and a platinum-group metal or a salt thereof supported on the carrier. This reaction can schematically be shown below.

$$2CO+2RONO \rightarrow \begin{array}{l} COOR \\ | \\ COOR \end{array} +2NO$$

(R=alkyl or cycloalkyl)

As the above scheme shows, this reaction

yields nitrogen monoxide equivalents to the consumed nitrous acid ester. Accordingly, the nitrogen monoxide thus formed may be recycled as the starting material for the above reaction by introducing an alcohol and a gas containing molecular oxygen to react them with the nitrogen monoxide as schematically shown below and recovering the resulting nitrous acid ester.

$$2NO+\frac{1}{2}O_2+2ROH\rightarrow 2RONO+H_2O$$

(R=alkyl or cycloalkyl)

An ester of nitrous acid with a saturated monohydric aliphatic alcohol having 1 to 8 carbon atoms or an alicyclic alcohol having 1 to 8 carbon atoms is preferred as the ester of nitrous acid. Examples of the aliphatic alcohol are methanol, ethanol, n-propanol, isopropanol, n-butanol, isobutanol, sec-butanol, tert-butanol, n-amyl alcohol, isoamyl alcohol, hexanol and octanol, and examples of the alicyclic alcohol include cyclohexanol, and methylcyclohexanol. These alcohols may contain a substituent, such as an alkoxy group, which does not inhibit the reaction.

The concentration of the ester of nitrous acid used may be varied over a wide range. To obtain a satisfactory rate of reaction, it is desirable to adjust the concentration of the nitrous acid ester in the starting gaseous mixture introduced into the reactor at 1% by volume or higher, for example about 5 to about 30% by volume.

Carbon monoxide used in the process of this invention may be pure or may be diluted with an inert gas such as nitrogen. The concentration of carbon monoxide in the reaction zone may be varied over a wide range and is usually in the range of 10 to 90% by volume.

The reaction is carried out under such conditions that no liquid phase is formed in the reaction zone (namely, in the gaseous or vapor phase). These conditions may vary depending upon the reaction temperature, the reaction pressure, the type and concentration of the nitrous acid ester, etc. Thus, these conditions may be properly selected so that the reaction is carried out in the vapor phase.

The reaction proceeds rapidly even at low temperatures, and side-reactions occurs less as the reaction temperature is lower. It is desirable therefore to perform the reaction at relatively low temperature at which the desired space time yield can be maintained, for example at a temperature of 50°C to 200°C, preferably at 80°C to 150°C. The reaction pressure can also be selected properly. For example, it is atmospheric pressure to 10 kg/cm$^2$.G, preferably atmospheric pressure to 5 kg/cm$^2$/G. Pressures below the above-specified lower limit, for example reduced pressures of down to 200 mmHg, can also be used.

The catalytic reaction in accordance with this invention may be carried out in a fixed or fluidized bed. The time of contact between the starting gaseous mixture and the catalyst can be properly chosen. For example, the contact time is not more than 20 seconds, preferably 0.2 to 10 seconds.

The nitrous acid ester can be prepared, for example, by reacting an alcohol with a nitrogen oxide in the optional presence of molecular oxygen. The reaction product gas contains the unreacted alcohol and nitrogen oxide (particularly nitrogen monoxide) and at times, traces of water and oxygen in addition to the desired nitrous acid ester. In the process of this invention, this product gas containing the nitrous acid ester can be used as the starting nitrous acid ester, and the platinum-group metal catalyst supported on silicon carbide exhibits excellent catalytic activity and high selectivity even when a nitrous acid ester containing such impurities is used.

The following Examples illustrate the present invention more specifically.

Example 1

(1) Preparation of a supported catalyst

Palladium chloride (1.12 parts by weight) was dissolved in 29.9 parts by weight of a 1.54% by weight aqueous solution of hydrochloric acid, and 30 parts by weight of silicon carbide having a purity of 85% (the remainder consisting of 11% of silica and 3% of alumina) having a specific surface area of less than 1 m$^2$/g was dipped in the solution. The solution was stirred at room temperature for about 2 hours.

The silicon carbide impregnated with palladium chloride was separated by decantation, dried, and then dipped in a solution consisting of 0.5 part by weight of sodium hydroxide, 0.7 part by weight of sodium hydrogen carbonate and 48.8 parts of water. The solution was stirred at about 70°C for about 9 hours to treat it with the alkali.

The alkali-treated product was washed with water until the washing became neutral and a chlorine ion was no longer detected. The washed product was then dipped in an aqueous hydrazine solution consisting of 3 parts by weight of 85% hydrazine hydrate and 97 parts by weight of water. The solution was stirred at room temperature for about 4 hours to perform the reducing treatment.

The treated product was decanted, washed with water and dried to give a spherical supported catalyst having a particle diameter of 3 mm and composed of silicon carbide and 0.56% by weight of palladium supported on it.

(2) Production of dimethyl oxalate

Ten milliliters (9.6g) of the supported catalyst prepared in (1) above was filled in a glass reaction tube having an inside diameter of 20 mm and a length of 55 cm, and glass beads

were further filled into the reaction tube and placed on the catalyst layer to a height of 20 cm.

The reaction tube was fixed vertically, and an annular electric heater was mounted on the outside of the reaction tube to maintain the temperature of the catalyst layer at 110°C.

From the top of the reaction tube, carbon monoxide, gaseous methyl nitrite, nitrogen monoxide, gaseous methanol and nitrogen were fed at a rate of 4, 3, 0.6, 3 and 9.4 Nl/hr, respectively, and reacted under atmospheric pressure.

The reaction product which left the reaction tube was passed through methanol to collect dimethyl oxalate. Low-boiling compounds not collected by methanol were then condensed by cooling with dry ice/methanol and collected. The liquids collected were each analyzed by gas chromatography. The results are shown in Table 1.

Example 2

A spherical catalyst having a particle diameter of 3 mm and composed of silicon carbide and 0.50% by weight of palladium supported on it was prepared in the same way as in Example 1, (1) except that silicon carbide having a purity of 90% (the remainder consisting of 7.3% of silica and 1.5% of alumina) and a specific surface area of 0.04 $m^2/g$ was used. Using 10 ml (8.4g) of the resulting catalyst, dimethyl oxalate was synthesized by the same procedure as in Example 1, (2). The results are shown in Table 1.

Comparative Examples 1 and 2

A spherical palladium catalyst supported on zeolite and having a particle diameter of 3 mm (Comparative Example 1) and a spherical palladium catalyst supported on silica (71%)-alumina (28%) (Comparative Example 2) were respectively prepared in the same way as in Example 1, (1). Using 10 ml (5.8g in Comparative Example 1 and 3.4g in Comparative Example 2) of each of these catalysts, dimethyl oxalate was synthesized in the same way as in Example 1, (2).

The results are shown in Table 1.

TABLE 1

| | Catalyst | | Amounts of products formed (mmol/hr) (*) | | | Space time yield of dimethyl oxalate (g/liter.hr) |
|---|---|---|---|---|---|---|
| | Carrier | Amount of Pd supported (wt.%) | Dimethyl oxalate | Dimethyl carbonate | Carbon dioxide | |
| Ex. 1 | Sic (85% pure) | 0.56 | 26.04 (98.4%) | 0.69 (1.3%) | 0.61 (0.3%) | 307 |
| Ex. 2 | SiC (90% pure) | 0.50 | 25.03 (98.2%) | 0.68 (1.3%) | 0.22 (0.4%) | 296 |
| Comp. Ex. 1 | Zeolite | 1.0 | 4.92 (94.3%) | 0.33 (2.7%) | 0.27 (2.2%) | 58 |
| Comp. Ex. 2 | Silica-alumina | 1.0 | 9.45 (91.6%) | 1.19 (5.8%) | 0.54 (2.6%) | 112 |

(*): The parenthesized figures in the middle columns are selectivities based on carbon monoxide.

Example 3

Ten milliliters (8.4g) of the same supported palladium catalyst as used in Example 2 was filled in a glass reaction tube having an inside diameter of 20 mm and a length of 55 cm, and glass beads were further filled into the reaction tube and placed on the catalyst layer to a height of 20 cm.

The reaction tube was fixed vertically, and an annular electric heater was mounted on the outside of the reaction tube to maintain the temperature of the catalyst layer at 110°C.

From the top of the reaction tube, a gaseous mixture consisting of 20% by volume of carbon monoxide, 15% by volume of methyl nitrite and 65% by volume of nitrogen was fed at a rate of 20 Nl/hr, and reacted under atmospheric pressure.

The reaction mixture was worked up in the same way as in Example 1, (2).

Dimethyl oxalate was obtained in an amount of 29.93 mmol/hr (space time yield: 354 g/liter.hr). Only 0.73 mmol/hr of dimethyl carbonate and 0.21 mmol/hr of carbon dioxide

were formed as by-products. The selectivity of dimethyl oxalate based on carbon monoxide was 98.45%.

Example 4

Ten milliliters (8.4g) of a spherical palladium catalyst having a particle diameter of 3 to 4 mm and composed of silicon carbide having a specific surface area of 0.04 $m^2/g$ and 0.5% by weight of palladium supported on it, which was prepared as in Example 1, (1), was filled in a jacketed glass reaction tube having an inside diameter of 20 mm and a length of 55 cm. Glass Raschig rings were further filled into the reaction tube and placed on the catalyst layer to a height of 20 cm. The reaction tube was fixed vertically, and a heat transfer medium was circulated through the jacket to maintain the temperature of the catalyst layer at 120°C.

From the top of the reaction tube, carbon monoxide, gaseous ethyl nitrite and nitrogen were fed at a rate of 24, 3.2 and 12.8 Nl/hr, respectively, and reacted under atmospheric pressure.

The product which left the reaction tube was passed through a condenser through which ice water was circulated. The liquid trapped by the condenser was analyzed by gas chromatography. It was found that diethyl oxalate was formed in an amount of 25.89 mmol/hr (space time yield: 378 g/liter.hr) and only 0.53 mmol/hr of diethyl carbonate was formed as a by-product. The selectivity of diethyl oxalate based on carbon monoxide was 98.99%.

Example 5

Ten milliliters (8.4g) of the same supported palladium catalyst as used in Example 2 was filled into a stainless steel reaction tube having an inside diameter of 23 mm and a length of 55 cm, and glass beads were further filled into it and placed on the catalyst layer to a height of 20 cm. The reaction tube was fixed vertically, and an annular electric heater was mounted on the outside of the reaction tube to maintain the temperature of the catalyst layer at 110°C.

From the top of the reaction tube, a gaseous mixture consisting of 20% by volume of carbon monoxide, 15% by volume of methyl nitrite, 3% by volume of nitrogen monoxide, 4% by volume of methanol and 58% by volume of nitrogen was fed into the reactor at a rate of 18.6 Nl/hr, and reacted under a pressure of 2.0 $kg/cm^2$.G.

The reaction product which left the reaction tube was passed through methanol to collect dimethyl oxalate. Low-boiling compounds not collected by methanol were then condensed by cooling with dry ice/methanol and collected. The liquids collected were each analyzed by gas chromatography.

It was found that dimethyl oxalate was obtained in a space time yield of 250 g/liter.hr, and the selectivities of dimethyl oxalate, dimethyl carbonate and carbon dioxide based on carbon monoxide were 95.4, 2.1 and 0.8%, respectively.

Example 6

The procedure of Example 5 was followed except that 10 ml (8.4g) of the same supported palladium catalyst as used in Example 2 was used, and a gaseous mixture consisting of 20% by volume of carbon monoxide, 9.2% by volume of methyl nitrite, 3% by volume of nitrogen monoxide, 2% by volume of methanol and 65.8% by volume of nitrogen was fed into the reaction tube from its top at a rate of 38.7 Nl/hr and reacted under a pressure of 4.6 $kg/cm^2$.G.

It was found that dimethyl oxalate was obtained in a space time yield of 241 g/liter.hr, and the selectivities of dimethyl oxalate, dimethyl carbonate and carbon dioxide gas based on carbon monoxide were 95.1, 2.4 and 2.5%, respectively.

Example 7

The procedure of Example 1 was followed except that 10 ml (9.6g) of a catalyst composed of silicon carbide and 0.51% by weight of palladium supported on it, which was prepared in the same way as in Example 1, (1), was used, and that the temperature of the catalyst layer was maintained at 120°C.

There were obtained 32.63 mmol/hr (space time yield: 385 g/liter.hr) of dimethyl oxalate , 0.51 mmol/hr of dimethyl carbonate and 0.29 mmol/hr of carbon dioxide gas. The selectivities of these products were 97.64, 1.93, and 0.43, respectively, based on carbon monoxide.

Example 8

(1) Preparation of a supported catalyst

Palladium chloride (9.43 parts by weight) was dissolved in 410 parts by weight of a 0.93% by weight aqueous solution of hydrochloric acid, and 400 parts by weight of silicon carbide having a specific surface area of less than 1 $m^2/g$ and a purity of 85% (the remainder consisting of 11% of silica and 3% of alumina) was dipped in the solution. The solution was stirred at room temperature for about 2 hours. The silicon carbide impregnated with palladium chloride was separated by decantation, air-dried and then dried at 120°C overnight.

The dried product (120 parts by weight) was dipped in 200 parts by weight of an aqueous solution containing 1% by weight of sodium hydroxide and 1.4% by weight of sodium hydrogen carbonate, and the solution was stirred at about 70°C for 4 hours to treat it with the alkalies. The alkali-treated product was then washed with warm water until the washing became neutral and a chlorine ion was no longer detected. The washed product was then dried overnight at 80°C.

The alkali-treated product was subjected to reducing treatment at 110°C for 1 hour in a gaseous mixture composed of 20% by volume of carbon monoxide, 15% by volume of methyl

nitrite, 3% by volume of nitrogen monoxide, 15% by volume of methanol and 47% by volume of nitrogen which can also be used as a starting material for synthesis of dimethyl oxalate. Thus, a catalyst composed of silicon carbide and 0.44% by weight of palladium supported on it was obtained.

(2) Production of dimethyl oxalate

Ten milliliters (9.6g) of the catalyst obtained in (1) above was filled into a jacketed glass reaction tube having an inside diameter of 20 mm and a length of 55 cm, and glass beads were further filled into it and placed on the catalyst layer to a height of 20 cm. The reaction tube was fixed vertically, and a heat transfer medium was circulated through the jacket to maintain the temperature of the catalyst layer at 110°C.

From the top of the reaction tube, carbon monoxide, gaseous methyl nitrite, nitrogen monoxide, gaseous methanol and nitrogen were fed into the reactor at a rate of 4, 3, 0.6, 3 and 9.8 Nl/hr, respectively, and reacted under atmospheric pressure.

The reaction product which left the reaction tube was passed through methanol to collect dimethyl oxalate. Low-boiling compounds not collected by methanol were condensed by cooling with dry ice/methanol and collected. The liquids collected were each analyzed by gas chromatography.

As a result, there were obtained dimethyl oxalate, dimethyl carbonate and carbon dioxide gas in an amount of 32.62 mmol/hr (space time yield 385 g/liter.hr), 2.77 mmol/hr, and 0.40 mmol/hr, respectively. The selectivities of these products based on CO were 95.37, 4.05, and 0.58%, respectively.

## Claims

1. A process for preparing a diester of oxalic acid by the vapor phase catalytic reaction of carbon monoxide and an ester of nitrous acid in the presence of a catalyst composed of a solid carrier and the platinum-group metal or a salt thereof supported on the carrier, characterized in that said solid carrier is silicon carbide.

2. The process of claim 1, characterized in that the silicon carbide carrier may contain up to 30% of silica, carbon, alumina, iron oxide, sodium oxide and/or potassium oxide.

3. The process of claim 1, characterized in that the ester of nitrous acid is an ester of nitrous acid with an alcohol having 1 to 8 carbon atoms selected from the group consisting of saturated monohydric aliphatic alcohols and alicyclic alcohols.

4. The process of claim 1, characterized in that the reaction is carried out at a temperature of 50°C to 200°C.

5. The process of claim 1, characterized in that the reaction is carried out under atmospheric pressure to 10 kg/cm².G.

6. The process of claim 1, characterized in that said catalyst is prepared by impregnating the silicon carbide carrier with an aqueous solution of a water-soluble salt of the platinum-group metal, treating the impregnated silicon carbide carrier with an alkali, and subjecting the alkali-treated product to reducing treatment in the liquid or gaseous phase.

7. The process of claim 5, characterized in that the alkali is a hydroxide, carbonate or bicarbonate of an alkali metal or an alkaline earth metal.

## Revendications

1. Procédé de préparation d'un diester de l'acide oxalique par réaction catalytique en phase vapeur de l'oxyde de carbone et d'un ester de l'acide nitreux, en présence d'un catalyseur constitué par un support solide et un métal du groupe du platine ou un sel de celui-ci fixé sur le support, caractérisé par le fait que ledit support solide est le carbure de silicium.

2. Procédé selon la revendication 1, caractérisé par le fait que le support de carbure de silicium peut contenir jusqu'à 30% de silice, de carbone, d'alumine, d'oxyde de fer, d'oxyde de sodium et/ou de potassium.

3. Procédé selon la revendication 1, caractérisé par le fait que l'ester de l'acide nitreux est un ester d'acide nitreux avec un alcool ayant 1 à 8 atomes de carbone choisi parmi le groupe des mono-alcools aliphatiques saturés et des alcools alicycliques.

4. Procédé selon la revendication 1, caractérisé par le fait que la réaction est effectuée à une température de 50°C à 200°C.

5. Procédé selon la revendication 1, caractérisé par le fait que la réaction est effectuée sous une pression comprise entre la pression atmosphérique et une pression manométrique de 10 kg/cm² (10 bars).

6. Procédé selon la revendication 1, caractérisé par le fait que ledit catalyseur est préparé en imprégnant le support de carbure de silicium avec une solution aqueuse d'un sel soluble dans l'eau du métal du groupe du platine, en traitant le support de carbure de silicium imprégné avec un produit alcalin et un soumettant le produit traité par le produit alcalin à un traitement réducteur dans la phase liquide ou gazeuse.

7. Procédé selon la revendication 5, caractérisé par le fait que le produit alcalin est un hydroxyde, un carbonate ou un bicarbonate d'un métal alcalin ou d'un métal alcalino-terreux.

## Patentansprüche

1. Verfahren zur Herstellung eines Oxalsäurediesters durch katalytische Dampfphasenreaktion von Kohlenmonoxid und einem Ester der salpetrigen Säure in Gegenwart eines Katalysators aus einem festen Träger und

einem sich auf dem Träger befindenden Metall der Platin-Gruppe oder einem Salz desselben, dadurch gekennzeichnet, dass der feste Träger Silicium-carbid darstellt.

2. Verfahren nach Anspruch 1, dadurch gekennzeichnet, dass der Siliciumcarbidträger bis zu 30% Siliciumdioxid, Kohlenstoff, Aluminiumoxid, Eisenoxid, Natriumoxid und/oder Kaliumoxid enthalten kann.

3. Verfahren nach Anspruch 1, dadurch gekennzeichnet, dass der Ester der salpetrigen Säure einen Salpetrigsäureester mit einem Alkohol mit 1 bis 8 Kohlenstoffatomen, ausgewählt aus der Gruppe der gesätigten einwertigen aliphatischen Alkohole und alicyclischen Alkohole, darstellt.

4. Verfahren nach Anspruch 1, dadurch gekennzeichnet, dass die Reaktion bei einer Temperatur von 50°C bis 200°C durchgeführt wird.

5. Verfahren nach Anspruch 1, dadurch gekennzeichnet, dass die Reaktion unter atmosphärischem Druck bis zu $10^6$ Pa durchgeführt wird.

6. Verfahren nach Anspruch 1, dadurch gekennzeichnet, dass der genannte Katalysator hergestellt wird durch Imprägnieren des Siliciumcarbidträgers mit einer wässrigen Lösung eines wasserlöslichen Metallsalzes der Platin-Gruppe, Behandeln des imprägnierten Siliciumcarbidträgers mit einem Alkali, und Reduktionsbehandlung des mit Alkali behandelten Produktes in der flüssigen oder gasförmigen Phase.

7. Verfahren nach Anspruch 5, dadurch gekennzeichnet, dass das Alkali ein Hydroxid, Carbonat oder Bicarbonat eines Alkalimetalles oder eines Erdalkalimetalles darstellt.